(19) Europäisches Patentamt
European Patent Office
Office européen des brevets

(11) Publication number: 0 231 080 B1

(12) EUROPEAN PATENT SPECIFICATION

(45) Date of publication of the patent specification: 19.04.89

(51) Int. Cl.4: C 11 D 3/00, C 11 D 3/48

(21) Application number: 87300364.4

(22) Date of filling: 16.01.87

# (54) Antiseptic compositions.

(30) Priority: 16.01.86 GB 8601048
27.03.86 GB 8607686

(43) Date of publication of application: 05.08.87 Bulletin 87/32

(45) Publication of the grant of the patent: 19.04.89 Bulletin 89/16

(84) Designated Contracting States: AT BE CH DE ES FR GB GR IT LI LU NL SE

(56) References cited:
CH-A-513 234
GB-A-1 338 003
US-A-4 330 531

(73) Proprietor: IMPERIAL CHEMICAL INDUSTRIES PLC, Imperial Chemical House Millbank, London SW1P 3JF (GB)

(72) Inventor: Bucknall, Robert Anthony, Endon Cottage 40 Higher Lane, Kerridge Macclesfield Cheshire (GB)
Inventor: Holloway, Philip Michael, 123 Abbey Road, Macclesfield Cheshire (GB)
Inventor: Platt, John Henry, 9 Somerset Close, Congleton Cheshire (GB)

(74) Representative: Arnold, Graham Donald, Imperial Chemical Industries PLC Legal Department: Patents PO Box 6, Welwyn Garden City Herts, AL7 1HD (GB)

EP 0 231 080 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

## Description

This invention relates to cleansing compositions, in particular cleansing compositions containing the antibacterial compound chlorhexidine or a salt thereof, which are suitable for skin cleansing and disinfection, .e.g. handwashing. The compositions of the invention nay therefore be useful in surgical practice, e.g. as a preoperative scrub, and they may also be used by hospital and medical staff in routine hygienic handwashing.

Our British Patent Specification No. 1 338 003 describes cleansing compositions comprising from 0.5 to 10 % by weight of a soluble salt of chlorhexidine, together with certain polyoxyethylene-polyoxypropylene block copolymers known as "Pluronic" surfactants, which are preferably present at 10 - 30 %, ideally 25; by weight of the composition. Further surfactants such as amine oxide foaming agents (e.g. lauryldimethylamine oxide) are preferably present in the composition at about 3.75 % by weight so that the total surfactant concentration is ideally about 28.75 %. The compositions of the said British Patent Specification may also contain perfumes colouring agents and as preservatives, isopropyl alcohol, ethyl alcohol, methyl p-hydroxybenzoate and propyl p-hydroxybenzoate.

We have now found that the antibacterial effect of compositions containing chlorhexidine salts may be increased by the use of a certain defined class of preservatives which generally have a synergistic effect in combination with chlorhexidine salts, and that in order to obtain the optimum antibacterial effect from the compositions of the invention they should contain a reduced amount of surfactant compared to the compositions of British Patent Specification 1 338 003.

Accordingly the invention provides an antiseptic composition suitable for use in skin cleansing and disinfection, e.g. handwashing, comprising;

(i) a chlorhexidine salt;
(ii) an aromatic alcohol = with the exception of methyl p-hydroxybenzoate or propyl p-hydroxy benzoate;
(iii) 10 % or less by weight of surfactant; and
(iv) an inert diluent or carrier.

It is desirable that the compositions should contain a salt of chlorhexidine which is soluble to the extent of at least 0.5 % w/v in water at ambient temperature. Suitable such salts are, for example, those formed with gluconic, 2-hydroxyethanesulphonic, formic, acetic, glutamic, succinnic, diglycolic, methanesulphonic, lactic, isobutyric and glucoheptonic acids and of these, the digluconate is particularly preferred.

The chlorhexidine salt is preferably pesent at a concentration of 0.1 to 10 % by weight of the composition, advantageously about 1 - 4 %.

The aromatic alcohol may be for example, a compound of the formula:

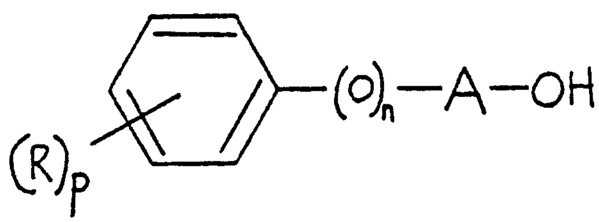

(I)

wherein

R is a (1 - 4C) alkyl group or a halogen atom,

A is a (1 - 4C) alkylene group,

p is 0 to 5 and

n is 0 or 1, for example a phenyl (1 - 4C) alkanol e.g. benzyl alcohol, 2-phenyl ethanol or 3-phenyl propanol or a phenoxy (1-4C) alkanol e.g. 2-phenoxyethanol. Derivatives of the above compounds wherein the benzene ring is substituted by 1 - 4C alkyl (e.g. methyl) or halo (e.g. chloro) substituents may also be suitable. Examples are p-chloro-2-phenoxyethanol and "Propylene Phenoxetol" available from Nipa Laboratories Limited.

The preferred aromatic alcohols are benzyl alcohol and 2-phenoxyethanol. 2-phenoxyethanol is particularly preferred.

The aromatic alcohol is preferably present at a minimum concentration of about 0.2 % by weight. The maximum concentration will be determined by the solubility of the alcohol, and will generally be not more than about 4 %. Preferably the concentration of alcohol will be about 1 - 2 %.

The surfactant may be of the type described in British Patent No. 1 338 003, i.e. non-ionic surfactants which are all members of a class of polyoxyethylene/polyoxypropylene block copolymers of the general formula:

$$HO\text{-}(CH_2\cdot CH_2\cdot O)_a(\underset{\displaystyle CH_3}{\underset{|}{CH}}\cdot CH_2\cdot O)_b(CH_2\cdot CH_2\cdot O)_cH$$

wherein a, b and c are integers, having molecular weights between 1000 and 16000, and in which the terminal polyoxyethylene chains represent 10 - 80 % of the molecule, which copolymers are available commercially under the trade name "Pluronic".

Particularly preferred "Pluronic"s are those with a polyoxypropylene typical molecular weight of about 2250, and containing 40 to 70 % of polyoxyethylene, that is "Pluronic"s P84, P85 and F87, which possess the optimum combination of foaming ability, mild detergency, viscosity, water solubility and non-irritancy. The Pluronic surfactant of choice is "Pluronic" F87.

Further surfactants described in British Patent Application 1 338 003 may be included, for example an amine oxide foaming agent. Suitable amine oxide foaming agents are, for example, cetyldimethylamine oxide, lauryldimethylamine oxide, cetylmethylmyristylamine oxide, dimethylmyristylamine oxide, and amidoalkyldimethylamine oxides e.g. $C_{7-17}$ fatty acid amidopropyldimethylamine oxides as sold by Th. Goldschmidt A. G. under the trade name "Aminoxid".

Other surfactants which may be used include betaine surfactants, for example fatty acid amidoalkyl-N-dimethylaminoacetic acid betaines for example $C_{7-17}$ fatty acid amidopropyl-N-dimethylaminoacetic acid betaines as sold by Th. Goldschmidt A. G. under the trade name "Tego-Betain".

Further sufactants which may be used include imidazoline derivatives, e.g. Monateric CA-35 available from Mona Industries, Inc; and alkanolamides e.g. monoand diethanolamides.

The above surfactants may be used singly or in combination, the total amount of surfactant in the composition being 10 % by weight or less, preferably less than about 5 % and advantageously less than about 2 %.

The inert diluent or carrier is conveniently water.

As certain surfactants e.g. those of the "Pluronic" type have, in addition to their surface activity, the effect of acting as "thickeners", it may be advantageous to add additional thickeners to compositions of the invention which contain no or little such surfactants. Suitable thickeners include high molecular weight polyethylene glycols, for example with molecular weight 15 - 20,000 (e.g. PEG 20,000). The concentration of thickener used will vary depending on the amount and type of surfactant used but may be for example from about 0 to 8 %, advantageously about 1 - 5 % by weight.

It may also be desirable for the compositions of the invention to contain additives ("foam enhancers") which enhance the quality of the foam or lather produced in use, e.g. its stability and 'feel'. Suitable such additives include those sold under the trade name "Glucam" (available from Amerchol) and low molecular weight (e.g. about 400) polyethylene glycols (e.g. PEG 400). Such additives are advantageously used at about 1 - 4 %, advantageously about 1 - 2 % by weight.

Because the present compositions contain a lower concentration of surfactant compared with those of British Patent 1 338 003 the present compositions may be of lower foamability. It may therefore be desirable to dispense the present compositions via a foam generating dispenser. Suitable such dispensers are described, for example, in published European Patent Applications Nos. 19 582 and 79 853.

Compositions according to the invention may also be formulated as gels. Such gels may contain increased amounts of thickeners and also may contain gelling agents.

It is also advantageous to adjust the pH of the compositions to between 5 and 8, to optimise the activity of the compositions. Suitable agents for adjusting the pH of the compositions are, for example, d-gluconolactone, sodium hydroxide solution or the acid from which the anion of the chlorhexidine salt in use is derived, e.g. gluconic acid. The compositions may also contain a buffering agent, e.g. sodium acetate.

The compositions may contain perfumes and colouring agents if required. These are not essential to the performance of the composition but may be desirable from the point of view of user acceptability.

The compositions may be prepared by adding the ingredients to purified water in any convenient order and mixing until the desired solution is obtained. It is generally convenient to add the chlorhexidine salt to the water first, followed by any solid components (e.g. PEG 20,000) which should be added with care to ensure even dispersion without formation of lumps. The surfactants and other ingredients are conveniently added next, followed by adjustment of the pH to the desired value. Finally further purified water is added if required to bring composition to the required dilution.

Table 1 demonstrates the advantageous antibacterial effects of mixtures of chlorhexidine digluconate and certain aromatic alcohols in comparison with corresponding mixtures with the preservatives mentioned in British Patent 1 338 003. The figures indicate the $log_{10}$ reduction of the population of Staph. aureus NCTC4163 upon exposure for one minute to (i) the preservative alone; (ii) chlorhexidine digluconate alone, and (iii) the mixture of chlorhexidine digluconate and the preservative. The test was performed in the presence of 10 % broth to simulate organic soiling. The proportions used are indicated in the figures in brackets following the name of the ingredient. The method used to obtain the figures is based on British Standard 3286 (1960).

One ml of a 24 hour tryptone soya broth (TSB) culture of Staph aureus NCTC4163 was added to 9 ml of the solution under test and a water control and thoroughly mixed. A 1 ml aliquot was removed after the appropriate time interval and immediately added to 9 ml of TSB containing 3 % azolectin and 20 % Tween 80 to neutralise the antiseptic carried over. Dilutions were then prepared from this in TSB containing 0.75 %

azolectin and 5 % Tween 80.

Viable counts were then performed on the test solution and water controls using pour-plate and spiral plating techniques. Tryptone soya agar (TSA) plates containing 0.3 % azalectin and 2 % Tween 80 to enable antiseptic neutralisation were used as growth medium. When chlorhexidine concentrations greater than 1 % were tested, 1 % suramin (Germanin, Bayer 205) was included in the pour-plate agar for additional neutralisation.

Tests were carried out prior to any study to ensure that the neutraliser system was adequate for the concentrations of chlorhexidine salts and alcohols tested.

From the viable counts, $\log_{10}$ survivor counts/ml of test solution were obtained, and by subtracting these from the water control counts, the $\log_{10}$ reduction in the population of the organisms achieved after the chosen contact time was determined.

**Table 1**

Bactericidal activity of various preservatives alone and in combination with chlorhexidine digluconate.

| Preservative | $\log_{10}$ reduction S. Aureus (NCTC4163) after one minute contact time | |
|---|---|---|
| | Preservative alone | Preservative +0.5 % chlorhexidine digluconate |
| chlorhexidine digluconate (0.5 %) (comparison) | 3.1 | -- |
| 2-phenoxyethanol (2 %) | <1.5 | >7.4 |
| 2-phenylethanol (2 %) | <1.5 | >7.4 |
| benzylalcohol (2 %) | <1.5 | >7.4 |
| "Propylene-Phenoxetol" (1 %) | <1.5 | >7.4 |
| p-chloro-2-phenoxyethanol | 2.3 | >7.4 |
| 3-phenylpropanol (2 %) | 4.1 | >7.4 |
| n-propanol (2 %) | <1.5 | 4.6 |
| iso-propanol (2 %) | <1.5 | 4.2 |
| methyl-hydroxybenzoate (0.1 %) | <1.5 | 4.4 |
| propyl-hydroxybenzoate (0.01 %) | <1.5 | 4.0 |
| ethanol (2 %) | <1.5 | 3.9 |

The invention is illustrated, but, not limited, by the following Examples.

**Example 1**

Handwash formulations were made up containing the following ingredients in the proportions indicated (by weight):

(1) Chlorhexidine digluconate 4 %;
(2) Preservative (identified in table);
(3) Pluronic F87 1.5 %;
(4) Lauryldimethylamine oxide 0.5 %;
(5) Polyethylene glycol 20,000 2 %

(6) Water (balance)

The compositions were tested in a formulation similar to that described in Example 1 of British Patent 1 338 003, but in which the proportions of (3) and (4) are reduced as indicated. In view of the low amount of Pluronic surfactant present, the polyethylene glycol 20,000 has been added as thickener. The perfume and colouring agents used in Example 1 of British Patent 1 338 003, which do not affect the performance of the composition in the test, have been omitted. The pH of the composition is adjusted to pH 5.5 with d-gluconolactone. The results are quoted in Table 2. It will be seen that the antibacterial activity of the formulations containing aromatic alcohols is much higher than that of the formulations containing isopropanol and ethanol. The 4 % isopropanol composition is directly comparable with Example 1 of British Patent 1 338 003. It can be seen that the compositions of the present invention are superior in performance to this composition.

**Table 2**

| Formulation (Preservative) | $Log_{10}$ reduction S. Aureus (NCTC4163) after one minute contact time |
|---|---|
| none (control) | 2.0 |
| Benzyl alcohol (2 %) | >7.2 |
| 2-Phenoxyethanol (2 %) | >7.2 |
| 2-Phenylethanol (2 %) | >7.2 |
| 3-Phenylpropanol (2 %) | >7.2 |
| Isopropanol (2 %) | 2.3 |
| Isopropanol (4 %) | 3.1 |
| Ethanol (2 %) | 2.4 |

**Examples 2 - 20**

The formulations specified in Table 3 were tested by the method described above but using the microorganism Staph. Aureus ATCC 6538. The $log_{10}$ reduction of the population of this organism after 1 minute contact time is given in Table 4. All the formulations show very good or excellent antibacterial activity.

**Table 3**

| Component (1) | % by weight in composition of Example No: | | | | | | | | | |
|---|---|---|---|---|---|---|---|---|---|---|
| | 2 | 3 | 4 | 5 | 6 | 7 | 8 | 9 | 10 | 11 |
| Chlorhexidine digluconate | 4 | 4 | 4 | 4 | 4 | 4 | 4 | 4 | 4 | 4 |
| PEG 20,000 | 2 | 2 | | 2 | 2 | 2 | 2 | - | - | - |
| Polymer JR 30M(2) | - | - | 0.1 | - | - | - | - | 0.1 | 0.1 | - |
| "Aminoxid" WS35(3) | 2 | 2 | 4 | 4 | - | 3 | 2 | 2 | - | - |
| "Tego-Betain" L5351(4) | 2 | 2 | - | - | 2 | 2 | 3 | 2 | - | - |
| PEG 400 | 1 | 1 | 1 | 1 | 1 | 2 | 2 | 1 | 1 | 1 |
| 2-phenoxyethanol | 2 | 2 | 2 | 2 | 2 | 2 | 2 | 2 | 2 | 2 |
| Sodium acetate | - | 2 | 2 | 2 | 2 | 2 | 2 | 2 | 2 | 2 |
| "Glucam" E20 | - | - | - | - | - | - | - | - | - | - |
| "Ammonyx" LO(5) | - | - | - | - | 2 | - | - | - | 2 | 2 |
| "Abil" B8834(6) | - | - | - | - | - | - | - | - | 2 | 2 |
| "Natrosol" 250HHR(7) | - | - | - | - | - | - | - | - | - | 0.08 |

Component (1) % by weight in composition of Example No:

| | 12 | 13 | 14 | 15 | 16 | 17 | 18 | 19 | 20 |
|---|---|---|---|---|---|---|---|---|---|
| Chlorhexidine digluconate | 4 | 4 | 4 | 4 | 4 | 4 | 2 | 2.5 | 4 |
| PEG 20,000 | - | 2 | 2 | 4 | 4 | 4 | 4 | 4 | 4 |
| Polymer JR 30M(2) | - | - | - | - | - | - | - | - | - |
| "Aminoxid" WS35(3) | - | 3 | 4.5 | 4 | 2.5 | 2.5 | 2.5 | 2.5 | 2.5 |
| "Tego-Betain" L5351(4) | - | 3 | 4.5 | 2 | 2.5 | 2.5 | 2.5 | 2.5 | 2.5 |
| PEG 400 | 1 | - | 2 | 4 | - | - | - | 1 | 1 |
| 2-phenoxyethanol | 2 | 2 | 2 | 2 | 1.5 | 1.5 | 1.5 | 1.5 | 1.5 |
| Sodium acetate | 2 | 2 | 2 | 2 | 2 | 2 | 2 | 2 | 2 |
| "Glucam" E20 | - | 2 | - | - | 2 | 2 | 2 | 1 | 1 |
| "Ammonyx" LO(5) | 4 | - | - | - | - | - | - | - | - |
| "Abil" B8834(6) | - | - | - | - | - | - | - | - | - |
| "Natrosol" 250HHR(7) | 0.08 | - | - | - | - | - | - | - | - |

**Notes**

1. Each composition additionally contains sufficient sodium hydroxide to bring the composition to pH 7, with the exception of Example 2, which contains sodium hydroxide to pH 5, and Example 11, which contains d-gluconolactone to pH 6. Each composition contains purified water to the balance of 100 %.
2. "Foam enhancer" , available from Union Carbide UK Ltd.
3. Supplied by Th. Goldschmidt A.G. at nominal concentration 35 %. Figure in table relates to amount of solution as supplied.
4. Supplied by Th. Goldschmidt A.G. at concentration 30 - 34 %. Figure in table relates to amount of solution as supplied.
5. Amine oxide foaming agent (lauryldimethylamine oxide) available from Millnaster Onyx UK, at concentration about 30 %. Figure in table relates to amount of solution as supplied.
6. "Foam enhancer"/emollient available from Th. Goldschmidt A.G.
7. "Foam enhancer"/thickener available from Hercules Ltd.

**Table 4**

Microbiological Results

| Example No. | $Log_{10}$ reduction S. Aureus ATCC 6538 in 1 min |
|---|---|
| 2 | 7.2 |
| 3 | >7.2 |
| 4 | >7.2 |
| 5 | >6.9 |
| 6 | >6.9 |
| 7 | >6.9 |
| 8 | >6.9 |
| 9 | >6.9 |
| 10 | 5.2 |
| 11 | >6.9 |
| 12 | >6.9 |
| 13 | >7.2 |
| 14 | >7.2 |
| 15 | >7.2 |
| 16 | >7.1 |
| 17 | >7.1 |
| 18 | 3.9 |
| 19 | >7.2 |
| 20 | >7.2 |

**Claims** for the Contracting states: BE, CH, DE, ES, FR, GB, IT, LI, LU, NL, SE

1. An antiseptic composition suitable for use in skin cleansing and disinfection comprising:

(i) a chlorhexidine salt;
(ii) an aromatic alcohol with the exception of methyl p-hydroxybenzoate or propyl p-hydroxy benzoate;
(iii) 10 % or less by weight of surfactant;
and
(iv) an inert diluent or carrier.

2. A composition as claimed in claim 1 wherein the chlorhexidine salt is soluble to the extent of at least 0.5 % w/v in water at ambient temperature.
3. A composition as claimed in claim 1 wherein the chlorhexidine salt is chlorhexidine digluconate.
4. A composition as claimed in any of claims 1 - 3 wherein the chlorhexidine salt is present at a concentration of from 1 to 4 % by weight of the composition.
5. A composition as claimed in any of claims 1 - 4 wherein the aromatic alcohol has the formula I

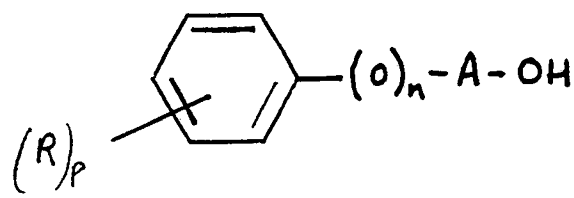

(I)

wherein R is a (1 - 4C)alkyl group or a halogen atom, A is a (1 - 4C)alkylene group, P is 0 to 5 and n is 0 or 1.
6. A composition as claimed in claim 5 wherein the aromatic alcohol is 2-phenoxyethanol or benzyl alcohol.
7. A composition as claimed in any preceding claim wherein the aromatic alcohol is present at a concentration of from 1 to 2 % by weight.
8. A composition as claimed in any preceding claim in which the surfactant is

(i) a polyoxyethylene/polyoxypropylene block copolymer of the general formula:

$$HO\text{-}(CH_2\cdot CH_2O)_a(\underset{\displaystyle CH_3}{\underset{|}{CH}}\cdot CH_2\cdot O)_b(CH_2\cdot CH_2\cdot O)_cH$$

wherein a, b and c are integers, having molecular weights between 1000 and 16000 and in which the terminal polyoxyethylene chains represent 10 - 80 % of the molecule;

(ii) an amine oxide foaming agent;
(iii) a betaine surfactant;
(iv) an imidazoline derivative;
(v) an alkanolamide;
or a mixture of any two or more of these.

9. A composition as claimed in any preceding claim in which the inert diluent or carrier is water.
10. A composition as claimed in any preceding claim which additionally contains one or more of the following ingredients:

(i) a thickener;
(ii) a foam enhancer;
(iii) a gelling agent;
(iv) a pH adjuster;
(v) a buffering agent;
(vi) a perfume; or
(vii) a colouring agent.

**Claims** for the contracting state: AT

1. A process for preparing an antiseptic composition suitable for use in skin cleansing and disinfection which comprises bringing into associations:

(i) a chlorhexidine salt;
(ii) an aromatic alcohol with the exception of methyl p-hydroxybenzoate or propyl p-hydroxy benzoate;
(iii) 10 % or less by weight of surfactant;
and
(iv) an inert diluent or carrier.

2. A process as claimed in claim 1 wherein the chlorhexidine salt is soluble to the extent of at least 0.5 % w/v in water at ambient temperature.
3. A process as claimed in claim 1 wherein the chlorhexidine salt is chlorhexidine digluconate.
4. A process as claimed in any of claims 1 - 3 for preparing a composition wherein the chlorhexidine salt is present at a concentration of from 1 to 4 % by weight of the composition.
5. A process as claimed in any of claims 1 - 4 wherein the aromatic alcohol has the formula I:

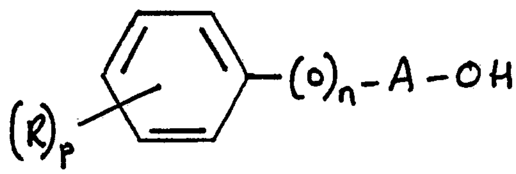

(I)

wherein R is a (1 - 4C)alkyl group or a halogen atom, A is a (1 - 4C)alkylene group, P is 0 to 5 and n is 0 or 1.
6. A process as claimed in claim 5 wherein the aromatic alcohol is 2-phenoxyethanol or benzyl alcohol.
7. A process as claimed in any preceding claim for preparing a composition wherein the aromatic alcohol is present at a concentration of from 1 to 2 % by weight,
8. A process as claimed in any preceding claim in which the surfactant is:

(i) a polyoxyethylene/polyoxypropylene block copolymer of the general formula:

$$HO\text{-}(CH_2\cdot CH_2\cdot O)_a(\underset{\displaystyle CH_3}{\underset{|}{CH}}\cdot CH_2\cdot O)_b(CH_2\cdot CH_2\cdot O)_cH$$

wherein a, b and c are integers, having molecular weights between 1000 and 16000 and in which the terminal polyoxyethylene chains represent 10 - 80 % of the molecule;

(ii) an amine oxide foaming agent;
(iii) a betaine surfactant;
(iv) an imidazoline derivative;
(v) an alkanolamide;
or a mixture of any two or more of these.

9. A process as claimed in any preceding claim in which the inert diluent or carrier is water.
10. A process as claimed in any preceding claim which additionally comprises bringing into association one or more of the following ingredients:

(i) a thickener;
(ii) a foam enhancer;
(iii) a gelling agent;
(iv) a pH adjuster;
(v) a buffering agent;
(vi) a perfume; or
(vii) a colouring agent.

**Patentansprüche** für den Vertragsstaat: AT

1. Verfahren zur Herstellung einer antiseptischen Zusammensetzung, die sich zur Verwendung bei der Hautreinigung und -desinfektion eignet, bei welchem folgendes zusammengebracht wird:

(i) ein Chlorhexidinsalz,
(ii) ein aromatischer Alkohol, mit Ausnahme von Methyl-p-hydroxybenzoat oder Propyl-p-hydroxybenzoat,
(iii) 10 Gew.-% oder weniger eines oberflächenaktiven Mittels
und
(iv) ein inertes Verdünnungs- oder Trägermittel.

2. Verfahren nach Anspruch 1, bei welchem das Chlorhexidinsalz in einem Ausmaß von mindestens 0,5 % (G/V) in Wasser bei Raumtemperatur löslich ist.

3. Verfahren nach Anspruch 1, bei welchem das Chlorhexidinsalz aus Chlorhexidindigluconat besteht.

4. Verfahren nach einem der Ansprüche 1 bis 3 zur Herstellung einer Zusammensetzung, bei welchem das Chlorhexidinsalz in einer Konzentration von 1 bis 4 Gew.-%, bezogen auf die Zusammensetzung, vorliegt.

5. Verfahren nach einem der Ansprüche 1 bis 4, bei welchem der aromatische Alkohol die Formel I:

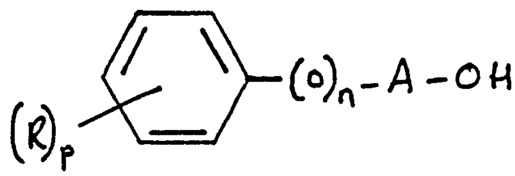
(I)

aufweist, worin R für eine (1 - 4C)Alkylgruppe oder ein Halogenatom steht, A für eine (1 - 4C)Alkylengruppe steht, p für 0 bis 5 steht und n für 0 oder 1 steht.

6. Verfahren nach Anspruch 5, bei welchem der aromatische Alkohol aus 2-Phenoxyethanol oder Benzylalkohol besteht.

7. Verfahren nach einem der vorhergehenden Ansprüche zur Herstellung einer Zusammensetzung, bei welcher der aromatische Alkohol in einer Konzentration von 1 bis 2 Gew.-% vorliegt.

8. Verfahren nach einem der vorhergehenden Ansprüche, bei welchem das oberflächenaktive Mittel aus folgendem besteht:

(i) einem Polyoxyethylen/Polyoxypropylen-Blockcopolymer der allgemeinen Formel

$$HO-(CH_2\cdot CH_2\cdot O)_a(\underset{\displaystyle CH_3}{\underset{|}{CH}}\cdot CH_2\cdot O)_b(CH_2\cdot CH_2\cdot O)_cH$$

worin a, b und c für Ganzzahlen stehen, das Molekulargewicht zwischen 1000 und 1600 liegt und die endständige Polyoxyethylenkette 10 bis 80 % des Moleküls ausmacht,

(ii) einem Aminoxidschäumungsmittel,
(iii) einem Betaintensid,
(iv) einem Imidazolinderivat,
(v) einem Alkanolamid
oder einem Gemisch aus zwei oder mehr derselben.

9. Verfahren nach einem der vorhergehenden Ansprüche, bei welchem das inerte Verdünnungs oder Trägermittel aus Wasser besteht.

10. Verfahren nach einem der vorhergehenden Ansprüche, bei welchem zusätzlich ein oder mehrere der folgenden Bestandteile eingemischt werden:

(i) ein Eindicker,
(ii) ein Schaumverstärker,
(iii) ein Gelierungsmittel,
(iv) ein pH-Justiermittel,
(v) ein Puffermittel,
(vi) ein Geruchsmittel oder
(v) ein Färbemittel.

**Patentansprüche** für die Vertragsstaaten: BE, CH, DE, ES, FR, GB, IT, LI, LU, NL, SE

1. Antiseptische Zusammensetzung für die Verwendung bei der Hautreinigung und -desinfektion, welche folgendes enthält:

(i) ein Chlorhexidinsalz,
(ii) einen aromatischen Alkohol, mit Ausnahme von Methyl-p-hydroxybenzoat oder Propyl-p-hydroxybenzoat)
(iii) 10 Gew.-% oder weniger eines oberflächenaktiven Mittels und
(iv) ein inertes Verdünnungs- oder Trägermittel.

2. Zusammensetzung nach Anspruch 1, bei welcher das Chlorhexidinsalz in einem Ausmaß von mindestens 0,5 % (G/V) in Wasser bei Raumtemperatur löslich ist.

3. Zusammensetzung nach Anspruch 1, bei welcher das Chlorhexidinsalz aus Chlorhexidindigluconat besteht.

4. Zusammensetzung nach einem der Ansprüche 1 bis 3, bei welcher das Chlorhexidinsalz in einer Konzentration von 1 bis 4 Gew.-%, bezogen auf die Zusammensetzung, vorliegt.

5. Zusammensetzung nach einem der Ansprüche 1 bis 4, bei welcher der aromatische Alkohol die Formel I

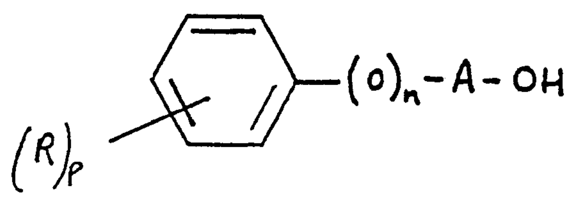

(I)

aufweist, worin R für eine (1 - 4C)Alkylgruppe oder ein Halogenatom steht, A für eine (1 - 4C)Alkylengruppe steht, p für 0 bis 5 steht und n für 0 oder 1 steht.

6. Zusammensetzung nach Anspruch 5, bei welcher der aromatische Alkohol aus 2-Phenoxyethanol oder Benzylalkohol besteht.

7. Zusammensetzung nach einem der vorhergehenden Ansprüche, bei welcher der aromatische Alkohol in einer Konzentration von 1 bis 2 Gew.-% vorliegt.

8. Zusammensetzung nach einem der vorhergehenden Ansprüche, bei welcher das oberflächenaktive Mittel aus folgendem besteht:

(i) einem Polyoxyethylen/Polyoxypropylen-Blockcopolymer der allgemeinen Formel

$$HO\text{-}(CH_2\cdot CH_2\cdot O)_a(\underset{\displaystyle CH_3}{\underset{|}{CH}}\cdot CH_2\cdot O)_b(CH_2\cdot CH_2\cdot O)_cH$$

worin a, b und c für Ganzzahlen stehen, das Molekulargewicht zwischen 1000 und 1600 liegt und die endständige Polyoxyethylenkette 10 bis 80 % des Moleküls ausmacht,

(ii) einem-Aminoxidschäumungsmittel,
(iii) einem Betaintensid,
(iv) einem Imidazolinderivat,
(v) einem Alkanolamid

oder einem Gemisch aus zwei oder mehr derselben.

9. Zusammensetzung nach einem der vorhergehenden Ansprüche, bei welcher das inerte Verdünnungs- oder Trägermittel aus Wasser besteht.

10. Zusammensetzung nach einem der vorhergehenden Ansprüche, welche zusätzlich einen oder mehrere der folgenden Bestandteile enthält:

(i) einen Eindicker,
(ii) einen Schaumverstärker,
(iii) ein Gelierungsmittel,
(iv) ein pH-Justiermittel,
(v) ein Puffermittel,
(vi) ein Geruchsmittel oder
(v) ein Färbemittel.

**Revendications** pour l'Etat contractant: AT

1. Procédé de préparation d'une composition antiseptique convenant pour l'utilisation dans le nettoyage et la désinfection de la peau, qui consiste à associer:

(i) un sel de chlorhexidine;
(ii) un alcool aromatique, à l'exception du parahydrorybenzoate de méthyle ou du para-hydrorybenzoate de propyle;
(iii) une quantité égale ou inférieure à 10 % en poids de surfactant; et
(iv) un diluant ou support inerte.

2. Procédé suivant la revendication 1, dans lequel le sel de chlorhexidine présente une solubilité d'au moins 0,5 % en poids/volume dans l'eau à température ambiante.

3. Procédé suivant la revendication 1, dans lequel le sel de chlorhexidine est le digluconate de chlorhexidine.

4. Procédé suivant l'une quelconque des revendications 1 à 3 pour la préparation d'une composition dans laquelle le sel de chlorhexidine est présent en une concentration de 1 à 4 % en poids de la composition.

5. Procédé suivant l'une quelconque des revendications 1 à 4, dans lequel l'alcool aromatique répond à la formule I:

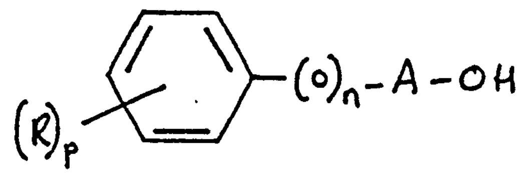

(I)

dans laquelle R représente un groupe alkyle en $C_1$ à $C_4$ ou un atome d'halogène; A représente un groupe alkylène en $C_1$ à $C_4$, p a une valeur de 0 à 5 et n a la valeur 0 ou 1.

6. Procédé suivant la revendication 5, dans lequel l'alcool aromatique est le 2-phénoxyéthanol ou l'alcool benzylique.

7. Procédé suivant l'une quelconque des revendications précédentes pour la préparation d'une composition dans laquelle l'alcool aromatique est présent en une concentration de 1 à 2 % en poids.

8. Procédé suivant l'une quelconque des revendications précédentes, dans lequel le surfactant est:

(i) un copolymère séquence polyoxyéthylène/polyoxypropylène de formule générale:

$$HO\text{-}(CH_2\cdot CH_2\cdot O)_a(\underset{\underset{CH_3}{|}}{CH}\cdot CH_2\cdot O)_b(CH_2\cdot CH_2\cdot O)_cH$$

dans laquelle a, b et c sont des nombres entiers, ayant un poids moléculaire compris dans l'intervalle de 1000 à 1600 et dans lequel les chaînes polyoxyéthylène terminales représentent 10 à 80 % de la molécule;

(ii) un agent moussant du type oxyde d'amine;
(iii) un surfactant du type bétaïne;
(iv) un dérivé d'imidazoline;
(v) un alcanolamide;
ou bien un mélange de deux ou plus de deux quelconques de ces composés.

9. Procédé suivant l'une quelconque des revendications précédentes, dans lequel le diluant ou support inerte est l'eau.

10. Procédé suivant l'une quelconque des revendications précédentes, qui consiste en outre à associer un ou plusieurs des ingrédients suivants:

(i) un épaississant;
(ii) un exalteur de mousse;
(iii) un agent gélifiant;
(iv) un agent d'ajustement du pH;
(v) un agent de tamponnement;
(vi) un parfum; ou bien
(v) un agent colorant.

**Revendications** pour les Etats contractants: BE, CH, DE, ES, FR, GB, IT, LI, LU, NL, SE

1. Composition antiseptique convenant pour l'utilisation dans le nettoyage et la désinfection de la peau, comprenant:

(i) un sel de chlorhexidine;
(ii) un alcool aromatique, à l'exception du parahydroxybenzoate de méthyle ou du parahydroxybenzoate de propyle;
(iii) une quantité égale ou inférieure à 10 % en poids d'un surfactant; et
(iv) un diluant ou support inerte.

2. Composition suivant la revendication 1, dans laquelle le sel de chlorhexidine présente une solubilité d'au moins 0,5 % en poids/volume dans l'eau à température ambiante.

3. Composition suivant la revendication 1, dans laquelle le sel de chlorhexidine est le digluconate de chlorhexidine.

4. Composition suivant l'une quelconque des revendications 1 à 3, dans laquelle le sel de chlorhexidine est présent en une concentration de 1 à 4 % en poids de la composition.

5. Composition suivant l'une quelconque des revendications 1 à 4, dans laquelle l'alcool aromatique répond à la formule I:

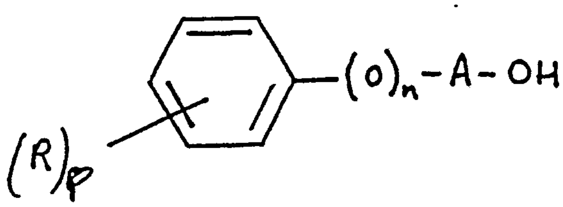

(I)

dans laquelle R représente un groupe alkyle en $C_1$ à $C_4$ ou un atome d'halogène, A représente un groupe alkylène en $C_1$ à $C_4$, p a une valeur de 0 à 5 et n a la valeur 0 ou 1.

6. Composition suivant la revendication 5, dans laquelle l'alcool aromatique est le 2-phénoxyéthanol ou l'alcool benzylique.

7. Composition suivant l'une quelconque des revendications précédentes, dans laquelle l'alcool aromatique est présent en une concentration de 1 à 2 % en poids.

8. Composition suivant l'une quelconque des revendications précédentes, dans laquelle le surfactant est:

(i) un copolymère séquence polyoxyéthylène/polyoxypropylène de formule générale:

$$HO\text{-}(CH_2\cdot CH_2\cdot O)_a(\underset{\displaystyle CH_3}{\underset{|}{CH}}\cdot CH_2\cdot O)_b(CH_2\cdot CH_2\cdot O)_cH$$

dans laquelle a, b et c sont des nombres entiers, ayant un poids moléculaire compris dans l'intervalle de 1000 à 1600 et dans lequel les chaînes polyoxyéthylène terminales représentent 10 à 80 % de la molécule;

(ii) un agent moussant du type oxyde d'amine;
(iii) un surfactant du type bétaïne;
(iv) un dérivé d'imidazoline;
(v) un alcanolamide;
ou bien un mélange de deux ou plus de deux quelconques de ces composés.

9. Composition suivant l'une quelconque des revendications précédentes, dans laquelle le diluant ou support inerte est l'eau.

10. Composition suivant l'une quelconque des revendications précédentes, qui contient en outre un ou plusieurs des ingrédients suivants:

(i) un épaississant;
(ii) un exalteur de mousse;
(iii) un agent gélifiant;
(iv) un agent d'ajustement du pH;
(v) un agent de tamponnement;
(vi) un parfum; ou
(v) un agent colorant.